# FASCICULE DE BREVET EUROPEEN

(11) **EP 0 341 141 B1**
(45) Date de publication et mention de la délivrance du brevet: **24.02.1993**
(21) Numéro de dépôt: 89401209.5
(22) Date de dépôt: 27.04.1989
(51) Int. Cl.: A43B 3/00, A43B 7/04

(54) **Chaussure à usage thérapeutique destinée à un traitement médical par application d'un champ ondulatoire électromagnétique**
Therapeutischer Schuh zur medizinischen Behandlung mittels eines elektromagnetischen Wellenfeldes
Therapeutic shoe for use in medical treatment by application of an undulating electro-magnetic field

(30) Priorité: 06.05.1988 FR 8806160
(43) Date de publication de la demande: 08.11.1989
(73) Titulaire: Bergery, Thierry, F-21160 Corcelles les Monts (FR); Chenut, Pascal, F-21220 Gevrey Chambertin (FR)
(72) Inventeur: Bergery, Thierry, F-21160 Corcelles les Monts (FR); Chenut, Pascal, F-21220 Gevrey Chambertin (FR)
(74) Mandataire: Bruder, Michel

(56) Documents cités:
- EP-A- 0 121 026
- DE-A- 2 175 100
- FR-A- 2 556 190

## Description

La présente invention concerne une chaussure à usage thérapeutique destinée à un traitement médical par application d'un champ ondulatoire électromagnétique.

On connaît à ce jour des moyens pour appliquer à des personnes dont certains organes sont malades, un traitement par une énergie ondulatoire tel qu'un champ magnétique ou des ondes électromagnétiques, par exemple des ondes hertziennes, sur des zones réflexes des pieds plus spécialement localisées à la plante des pieds. La couleur est un principe de traitement utilisé depuis fort longtemps par des civilisations anciennes. On sait d'autre part que les divers organes du corps humain sont reliés à des zones réflexes placées dans la zone cutanée des pieds et qu'en excitant les zones réflexes de la plante des pieds on peut obtenir la guérison de certaines maladies affectant les organes respectifs.

Les procédés utilisés à ce jour n'ont cependant pas eu le succès auquel on pouvait s'attendre et les dispositifs qui ont déjà été utilisés, tels que des semelles magnétiques, des patins ou des piédestaux connus à ce jour ne se sont pas avérés efficaces plus spécialement du fait qu'ils n'ont mis à profit ni toute la gamme adéquate d'ondes, ni tous les points du pied possédant de telles zones réflexes.

On connaît aussi, ainsi qu'il est décrit dans le document EP-A-0 121 026, une chaussure de sport éclairée, comportant une lampe montée sur la chaussure et qui est reliée électriquement, par des conducteurs, à une batterie logée dans la semelle de la chaussure. Cette chaussure à dispositif d'éclairage incorporé, permet d'augmenter la sécurité de son porteur.

La présente invention a pour objet une chaussure à usage thérapeutique destinée à un traitement médical par application d'un champ ondulatoire électromagnétique du type à ondes lumineuses, comprenant une semelle associée à un talon et constituée d'au moins trois couches superposées, à savoir une couche externe ou inférieure constituant la structure de base de la chaussure, un revêtement intermédiaire et une couche interne, et des lampes électriques reliées, par un réseau de conducteurs électriques, à des piles électriques, caractérisée en ce que chaque lampe est portée par une douille coaxiale ouverte à ses deux extrémités, noyée dans le matériau de la couche interne, la face supérieure de chaque lampe étant mise à découvert afin d'envoyer la lumière émise vers une zone réflexe du pied, le réseau de conducteurs électriques est monté sur la face du revêtement intermédiaire qui est dirigée vers l'intérieur, ce réseau étant composé d'une série de barres étroites parallèles s'étendant dans le sens longitudinal de la chaussure, constituant deux peignes à dents imbriquées alternativement mais ne se touchant pas, chaque douille étant de dimension telle qu'elle chevauche deux barres conductrices de polarités différentes pour l'alimentation de la lampe se trouvant dans la douille, les lampes électriques étant réparties de manière à exciter, par leurs rayonnements lumineux, toutes les zones réflexes des pieds afférentes aux divers organes du corps humain nécessitant un traitement médical.

L'expérience a montré qu'une telle chaussure donne les meilleurs résultat quant à l'efficacité du traitement, ce qui en fait un moyen hautement salutaire à la disposition des médecins, des podologues, des kinésithérapeutes et des ostéopathes.

On décrira ci-après, à titre d'exemple non limitatif, une forme d'exécution de la présente invention, en référence au dessin annexé sur lequel :

La figure 1 est une vue en coupe verticale et longitudinale d'une chaussure de l'invention.

La figure 2 est une vue en plan schématique de la semelle de la chaussure de l'invention vue du côté du réseau de conducteurs électriques monté sur la face dirigée vers l'intérieur du revêtement de la couche externe.

La figure 3 est une vue partielle, de détail, à plus grande échelle et en coupe de la semelle de la chaussure selon l'invention.

La chaussure 1 à usage thérapeutique suivant l'invention est destinée à un traitement médical par application d'un champ ondulatoire électromagnétique du type ondes lumineuses engendrées par des sources lumineuses telles que des lampes électriques alimentées en énergie par des piles électriques de très faible tension.

Ces lampes électriques sont disposées de manière à exciter, par leurs rayonnements, toutes les zones réflexes des pieds afférentes aux divers organes du corps humain et plus spécialement aux divers organes malades qui nécessitent un traitement médical.

La chaussure 1 comprend une semelle 2, un rebord 3 et un dessus chaussant 4. La semelle 2 est associée à un talon 5. Elle est constituée d'au moins trois couches superposées, associées de manière généralement connue. Ces trois couches sont constituées par une couche externe ou inférieure 6 constituant la structure de base de la chaussure ensuite vers l'intérieur de la chaussure un revêtement 7 intermédiaire, puis encore du côté intérieur une couche interne 8 en une matière plastique semi-dure, permettant un usage agréable de la chaussure proposée.

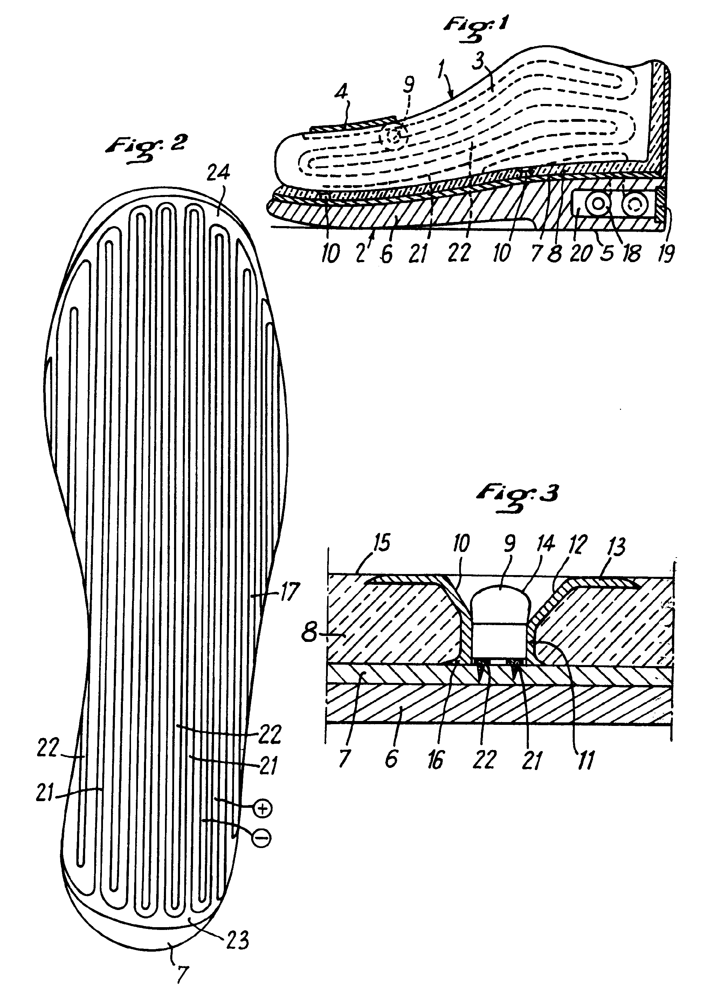

Elle comporte ensuite, du côté intérieur, des lampes 9 constituant les sources de lumière de traitement. Ces lampes 9 sont portéeschacune par une douille coaxiale 10, de forme générale tubulaire, ouverte à ses deux extrémités, composée d'une partie inférieure cylindrique 11, d'une partie intermédiaire tronconique 12 pour servir de diffuseur à la lumière émise par la lampe 9 et d'une partie supérieure 13 en forme de collerette. Cette douille 10 est noyée dans le matériau de la couche interne 8 afin d'être fixée et retenue en place au droit de la zone réflexe qui doit être soumise au traitement médical par chromothérapie selon l'invention. La face supérieure 14 de la lampe 9, mise à découvert afin d'envoyer la lumière émise vers une zone réflexe du pied, est de préférence, lorsque la lampe est en place, plus basse que le niveau supérieur de la face supérieure 15 de la couche interne 8 de la semelle, afin de ne pas gêner la marche. La douille 10 comporte, à son extrémité inférieure, des becs de retenue 16, orientés vers l'extérieur et attenants à la partie inférieure cylindrique 11, pour empêcher la douille de se détacher de la couche interne 8 dont elle est ainsi rendue solidaire. En dessous de la douille 10 est monté, sur la face dirigée vers l'intérieur du revêtement 7 de la couche externe 6 de la semelle, un réseau de conducteurs électrique, 17 servant à alimenter les lampes 9 en courant électrique provenant de piles électriques 18 de faible tensionn. Ces piles 18 sont logées de préférence dans un creux 20 aménagé dans le talon 5 de la chaussure 1, fermé vers l'extérieur par une porte coulissante 19. Le réseau de conducteurs 17 est composé de préférence d'une série de barres étroites parallèles 21,22, s'étendant dans le sens longitudinal de la chaussure 1. Ces barres sont reliées alternativement, en forme de peigne, par deux barres transversales 23,24, placées l'une du côté du talon 5 et l'autre du côté de la pointe de la chaussure 1. Les barres étroites 21,22, constituant deux peignes à dents imbriquées alternativement, mais ne se touchant pas, sont branchées à l'aide des barres transversales 23,24 prolongées, aux piles 18. Plus spécialement le peigne de barres 21 est relié au pôle + et celui des barres 22 est relié au pôle - des piles électriques 9. Les douilles 10 sont de dimension telle qu'elles chevauchent chacune, ainsi que montré sur la figure 3 du dessin, deux barres 21 et 22 de polarités différentes, de sorte que la lampe 9 respective soit alimentée correctement à partir des piles 18. De préférence, le réseau 17 de conducteurs électriques d'alimentation des lampes 9 est prolongé sur le rebord 3 de la chaussure 1, ainsi que montré schématiquement en traits pointillés sur la figure 2 du dessin, afin que tous les points constituant des zones réflexes podales puissent être pourvus de lampes 9 d'irradiation. Le circuit d'alimentation des lampes 9 peut être pourvu d'un interrupteur de coupure d'alimentation, afin d'économiser les piles 9 si besoin est. On peut également sectionner au moins l'un des peignes du réseau 17, afin de pourvoir l'ensemble d'alimentation des lampes 9 de moyens d'utilisation partielle de l'ensemble d'irradiation des zones réflexes podales. Ces moyens n'ont pas été figurés sur le dessin afin de le simplifier.

## Revendications

1. Chaussure à usage thérapeutique destinée à un traitement médical par application d'un champ ondulatoire électromagnétique du type à ondes lumineuses, comprenant une semelle (2) associée à un talon (5) et constituée d'au moins trois couches superposées, à savoir une couche externe ou inférieure (6) constituant la structure de base de la chaussure, un revêtement intermédiaire (7) et une couche interne, et des lampes électriques (9) reliées, par un réseau de conducteurs électriques (17), à des piles électriques (18), caractérisée en ce que chaque lampe (9) est portée par une douille coaxiale (10) ouverte à ses deux extrémités, noyée dans le matériau de la couche interne (8), la face supérieure (14) de chaque lampe (9) étant mise à découvert afin d'envoyer la lumière émise vers une zone réflexe du pied, le réseau de conducteurs électriques (17) est monté sur la face du revêtement intermédiaire (7) qui est dirigée vers l'intérieur, ce réseau étant composé d'une série de barres étroites parallèles (21,22) s'étendant dans le sens longitudinal de la chaussure, constituant deux peignes à dents imbriquées alternativement mais ne se touchant pas, chaque douille (10) étant de dimension telle qu'elle chevauche deux barres conductrices (21,22) de polarités différentes pour l'alimentation de la lampe (9) se trouvant dans la douille, les lampes électriques (9) étant réparties de manière à exciter, par leurs rayonnements lumineux, toutes les zones réflexes des pieds afférentes aux divers organes du corps humain nécessitant un traitement médical.

2. Chaussure suivant la revendication 1 caractérisée en ce que chaque douille (10) est composée d'une partie inférieure cylindrique (11), d'une partie intermédiaire tronconique (12) et d'une partie supérieure (13) en forme de collerette (9) et elle est pourvue de becs de retenue (16) attenants à la partie inférieure cylindrique (11) et orientés vers l'extérieur.

3. Chaussure suivant l'une quelconque des revendications 1 et 2 caractérisée en ce que les barres conductrices longitudinales (21,22) sont reliées alternativement par deux barres transversales (23,24), placées l'une du côté du talon (5) et l'autre du côté de la pointe de la chaussure (1), les barres conductrices (21,22) étant reliées aux piles (18) à l'aide des barres transversales (23,24) prolongées.

4. Chaussure suivant l'une quelconque des revendications 1 à 3 caractérisée en ce que le réseau (17) de conducteurs électriques d'alimentation des lampes (9) est prolongé sur le rebord (3) de la chaussure (1).

5. Chaussure suivant l'une quelconque des revendications 1 à 4 caractérisée en ce que les piles (18) sont logées dans un creux (20) aménagé dans le talon (5) de la chaussure (1), fermé vers l'extérieur par une porte coulissante (19).

6. Chaussure suivant l'une quelconque des revendications précédentes caractérisée en ce que le circuit d'alimentation des lampes (9) est pourvu d'un interrupteur de coupure d'alimentation.

7. Chaussure suivant l'une quelconque des revendications 1 à 6 caractérisée en ce qu'au moins l'un des peignes du réseau de conducteurs (17) est pourvu de moyens d'utilisation partielle des lampes.

## Patentansprüche

1. Therapeutischer Schuh zur medizinischen Behandlung mittels eines elektromagnetischen Wellenfeldes mit Lichtwellen, welcher eine Sohle (2) mit einem Absatz (5) aufweist und welcher von wenigstens drei übereinanderliegenden Schichten umfassend eine äußere oder untere Schicht (6) gebildet wird, welche das Unterteil des Schuhs bildet, ferner umfassend eine Zwischenschicht (7) und eine innere Schicht, und welcher ferner elektrische Lampen (9) aufweist, welche über ein Netz (17) von elektrischen Leitungen mit einer Batterieversorgung (18) verbunden sind, **dadurch gekennzeichnet,** daß jede Lampe (9) von einer koaxialen Buchse (10) getragen wird, welche an ihren beiden Enden offen ist, und die in das Material der inneren Schicht (8) eingelassen ist, die obere Fläche (14) jeder Lampe (9) freilegbar ist, um das abgegebene Licht auf eine Reflexzone des Fußes zu richten, das Netz (17) von elektrischen Leitern auf der Fläche der Zwischenschicht (7) angebracht ist, welche zur Innenseite weist, daß das Netz von einer Gruppe von schmalen, parallelen Stäben (21, 22) gebildet wird, welche sich in Längsrichtung des Schuhs erstrecken, und zwei mit Zinken versehene Kämme bilden, welche wechselseitig ineinandergreifen sich aber nicht berühren, jede Buchse (10) solche Abmessungen hat, daß sich zwei leitende Stäbe (21, 22) mit unterschiedlicher Polarität zur Versorgung der Lampe (9) überlappen und sich in der Buchse befinden, und daß die elektrischen Lampen (9) derart verteilt sind, daß über ihre Lichtstrahlen alle Reflexzonen des Fußes, welche mit diversen Organen des menschlichen Körpers zusammenhängen, und welche medizinisch zu behandeln sind, erregt werden.

2. Schuh nach Anspruch 1, **dadurch gekennzeichnet,** daß die Buchse (10) einen unteren zylindrischen Abschnitt (11), einen kegelstumpfförmigen Zwischenabschnitt (12) und einen oberen Abschnitt (13) in Form eines Bundes (9) umfaßt, und daß sie mit einem Haltebund (16) versehen ist, welcher sich an den unteren zylindrischen Abschnitt (11) anschließt und nach außen weist.

3. Schuh nach einem der Ansprüche 1 und 2, **dadurch gekennzeichnet,** daß die leitenden Längsstäbe (21, 22) wechselweise über zwei Querstäbe (23, 24) verbunden sind, welche einerseits auf der Seite des Absatzes und andererseits auf der Seite des Zehenteils des Schuhs (1) angeordnet sind, und daß die leitenden Stäbe (21, 22) mit der Batterie (18) mit Hilfe von verlängerten Querstäben (23, 24) verbunden sind.

4. Schuh nach einem der Ansprüche 1 bis 3, **dadurch gekennzeichnet,** daß das Netz (7) aus elektrischen Versorgungsleitern für die Lampen (9) über den Rand (3) des Schuhs (1) hinaus verlängert ist.

5. Schuh nach einem der Ansprüche 1 bis 4, **dadurch gekennzeichnet,** daß die Batterie in einer Ausnehmung (20) untergebracht ist, welche im Absatz (15) des Schuhs (1) vorgesehen ist und die nach außen hin mittels einer Schiebetüre (19) geschlossen ist.

6. Schuh nach einem der vorangehenden Ansprüche, **dadurch gekennzeichnet,** daß die Versorgungsschaltung für die Lampen (9) mit einem Versorgungsunterbrechungsschalter versehen ist.

7. Schuh nach einem der Ansprüche 1 bis 6, **dadurch gekennzeichnet,** daß wenigstens einer der Kämme des Leiternetzes (17) mit Einrichtungen zur teilweisen Nutzung der Lampen versehen ist.

## Claims

1. Shoe for therapeutical use intended for medical treatment by application of an electromagnetic undulatory field of the type employing light waves, comprising a sole (2) associated with a heel (5) and constituted by at least three superposed layers, namely an outer or lower layer (6) constituting the base structure of the shoe, an intermediate coating (7) and an inner layer, and electric lamps (9) connected, by a network of electrical leads (17), to electric batteries (18), characterized in that each lamp (9) is borne by a coaxial socket (10) open at its two ends, embedded in the material of the inner layer (8), the upper face (14) of each lamp (9) being uncovered in order to send the light emitted towards a reflex zone of the foot, the network of electrical leads (17) is mounted on that face of the intermediate coating (7) which is directed inwardly, this network being composed of a series of parallel narrow bars (21, 22) extending lengthwise of the shoe, constituting two combs with alternately imbricated teeth but not touching, each socket (10) being of such dimensions that it overlaps two conducting bars (21, 22) of different polarities for the supply of the lamp (9) located in the socket, the electric lamps (9) being distributed so as to energize, by their light rays, all the reflex zones of the feet corresponding to the various organs of the human body requiring medical treatment.

2. Shoe according to Claim 1, characterized in that each socket (10) is composed of a lower cylindrical part (11), a truncated intermediate part (12) and an upper part (13) in the form of a flange (9) and it is provided with retaining beaks (16) contiguous with the cylindrical lower part (11) and oriented outwardly.

3. Shoe according to either one of Claims 1 and 2, characterized in that the longitudinal conducting bars (21, 22) are alternately connected by two transverse bars (23, 24) placed, one, towards the heel (5) and the other, towards the tip of the shoe (1), the conducting bars (21, 22) being connected to the batteries (18) with the aid of the extended transverse bars (23, 24).

4. Shoe according to any one of Claims 1 to 3, characterized in that the network (17) of electrical leads for supplying the lamps (9) is extended on the raised side (3) of the shoe (1).

5. Shoe according to any one of Claims 1 to 4, characterized in that the batteries (18) are housed in a recess (20) made in the heel (5) of the shoe (1), closed on the outside by a sliding door (19).

6. Shoe according to any one of the preceding Claims, characterized in that the circuit for supplying the lamps (9) is provided with a switch for cutting off supply.

7. Shoe according to any one of Claims 1 to 6, characterized in that at least one of the combs of the network of conductors (17) is provided wits means for partial use of the lamps.
